# EUROPEAN PATENT APPLICATION

(11) **EP 4 091 708 A1**
(43) Date of publication of application: **23.11.2022**
(21) Application number: 22173994.9
(22) Date of filing: 18.05.2022
(51) Int. Cl.: B01J 3/04, B01J 19/00, C07D 251/60, C07D 251/62

(54) **APPARATUS FOR THE TREATMENT OF GASEOUS STREAMS LEAVING THE REACTION SECTION IN A PLANT FOR THE PRODUCTION OF MELAMINE AND CORRESPONDING PROCESS**

(30) Priority: 20.05.2021 IT 202100013088
(71) Applicant: EUROTECNICA MELAMINE AG, 8832 Wollerau (CH)
(72) Inventor: DE AMICIS, Alberto, I-20097 San Donato Milanese (MI) (IT); DI RUOCCO, Giuseppe, I-23900 Lecco (IT); SANTUCCI, Roberto, I - 21050 Gorla Maggiore (VA) (IT)
(74) Representative: De Gregori, Antonella

(57) **Abstract**

An apparatus is described for the treatment of gaseous streams leaving the reaction section in a plant for the production of melamine together with the corresponding process.

## Description

The present invention relates to an apparatus for treating the gaseous streams leaving the reaction section in a plant for the production of melamine and the corresponding process.

For a better understanding of the object of the present invention, reference is made to the state of the art as described hereunder.

It is known that the transformation of molten urea into melamine is described by the following overall reaction (1):

6 NH₂CONH₂ → (CN)₃(NH₂)₃ + 6 NH₃ + 3 CO₂ (1)

urea melamine ammonia carbon dioxide
wherein, for every kilogram of melamine, 1.86 kilograms of NH₃ and CO₂, globally called off-gases, are formed.

As has been described numerous times, the off-gases coming from the conversion reaction of urea into melamine are recycled to a urea plant for their conversion and therefore represent a supply stream to the urea plant that contains NH₃ and CO₂.

The reaction of urea production from NH3 and CO₂ takes place through the following reaction:

2 NH₃ + 1 CO₂ → 1 CO(NH₂)₂ + 1 H₂O (2)

ammonia carbon dioxide urea water

The molar ratio between NH3 and CO₂ is equal to 2 and this ratio is that which is optimal for all the supply streams to the urea plant, including the off-gas stream coming from the melamine plant.

The ratio between NH₃ and CO₂ in the off-gas stream from the melamine plant depends on how much ammonia is fed to the melamine reaction section.

As can be seen from reaction (1) above, also in this case the NH₃ and CO₂ ratio is equal to 2 and this ratio is modified by the quantity of NH3 that is fed into the bottom of the reactor and post reactor.

As described hereunder, it is important to feed NH₃ to the reactor and post-reactor that form the reaction section of the apparatus for the production of melamine, in order to favour the conversion of the by-products to melamine in the reactor and strip the CO₂ from the raw melamine in the post-reactor. It is consequently always necessary, for the requirements of the melamine plant, to maintain a stream of NH₃ in the feeding to the reaction section.

It should also be remembered that in urea technologies, the supply of NH3 and CO₂ to the urea reactor does not have a stoichiometric ratio of 2, but rather a ratio that varies according to the technologies themselves. All the technologies used industrially for the production of urea are with total recycling and, regardless of the NH3/CO2 ratio in the reactor, the NH3/CO2 ratio at the inlet of the plant is equal to 2. In order to satisfy the technological necessity of the urea reactor of operating with an NH₃/CO₂ molar ratio higher than the stoichiometric ratio, a series of recyclings between the different sections are applied in the different technologies in order to recycle the reagents in the reactor until they are converted into urea. In order to be optimal for the requirements of the urea plant, the off-gas stream from the melamine plant must have an NH3/CO2 ratio as close as possible to the stoichiometric ratio in order to limit the number of recyclings inside the urea plant.

Almost all of the latest-generation melamine plants, operating at high pressure with a pressure higher than 70 barg and a temperature above 360°C, adopt a reaction section comprising at least one reactor and at least one post-reactor (also called finishing converter or finisher or NH₃ stripper), from each of which at least two streams are extracted, a stream in gaseous phase composed of NH3, CO₂ and melamine in vapour phase and a stream of molten melamine, comprising by-products and reaction intermediates, and in which NH3 and CO₂ remain in solution, in equilibrium with their partial pressures.

In some plants, a solution is preferred which provides a single outlet from the reactor, which is equipped, downstream, with a separator, from which the above-mentioned gas and liquid phases exit separately.

The gaseous streams exiting from the upper portion of the reactor and post-reactor, before being used in other processes, for example before being used for the production of urea, must be treated in order to separate and recover the melamine present in the same, at the same time recovering at least part of the heat contained therein.

There are mainly two methods used for this washing treatment (also called scrubbing): scrubbing the gaseous streams with aqueous solutions or scrubbing the gaseous streams with molten urea. After scrubbing, once the traces of melamine have been removed, the gaseous streams are called off-gases.

This scrubbing step does not create problems in the case of scrubbing with aqueous solutions as
- the heat contained in the gaseous streams is used for maintaining the operating temperature of the system and
- the aqueous scrubbing solution is always available as long as the sections downstream of the reaction section are running and
- the scrubbing solution itself can be recycled for a long time as it is not subject to degradation or saturation of melamine as this is continuously extracted in the sections downstream (i.e. in the crystallization sections).

In the case of scrubbing with molten urea, on the contrary, various problems must be considered: as molten urea is in fact subject to degradation reactions with the formation of by-products which, above certain concentrations, precipitate with the formation of a solid product and with the consequent clogging of the system, and as the degradation rate of urea is directly proportional to the temperature, the temperature must be controlled by recycling through a heat exchanger with the production of steam to prevent the urea, on excessivley increasing the temperature, from rapidly degrading with the formation of by-products. The temperature is normally kept at around 180°C:
A further drawback of scrubbing with urea is that the quantity of melamine that may be present in dispersion in the molten scrubbing urea is limited by the necessity of avoiding its possible precipitation and the formation of encrustations.

It is consequently evident that the scrubbing urea cannot be kept in recycling without adequate renewal to avoid its saturation of both melamine and by-products.

It is evident that the urea used for scrubbing the gaseous streams leaving the reaction section, containing melamine, can only be used for the production of melamine, therefore it can only be recycled as a feed to the reactor for the synthesis of melamine: the quantity of urea that can be used for continuously renewing the urea circulating in the scrubbing section (i.e. the scrubbing urea) therefore corresponds to the quantity of urea used for scrubbing which is fed to the reactor. In the event of a stoppage in the supply of urea to the reactor, there is therefore no possibility of renewing the scrubbing urea used for scrubbing the gas phases.

According to the state of the art, the two gaseous streams leaving the reactor and the post-reactor respectively are generally combined and scrubbed in a single scrubbing column in order to recover the melamine contained therein and then sent to plants downstream for their recovery. The off-gases are normally sent to a urea plant as they mainly consist of NH₃ and CO₂.

The scrubbing operation is of primary importance not only for the recovery of the melamine contained in the gaseous streams, but above all for preventing said melamine in the vapour phase, when not adequately absorbed and dispersed, or solubilized in the scrubbing medium, from exiting from the reaction section which is kept at temperatures higher than 360°C (preferably higher than 370°C), then condensing in cold areas of the system, causing progressive clogging.

It therefore appears essential that the gaseous streams exit from the reactor and post-reactor in the presence of the scrubbing fluid suitable for separating and absorbing the melamine contained therein. This aspect represents a considerable limitation in the case of scrubbing effected with urea as, even during the transitional phases in which no urea is fed to the reaction section, but the reactor and post-reactor are kept full for a rapid restart, NH3 must be fed to the reaction section. There is in any case a gas phase containing melamine that must be scrubbed and this operation cannot take place in the scrubbing column with preselected urea, as the scrubbing fluid is lacking, but resort must be made to alternative methods or the reaction section must be rapidly emptied. These transitory phases in which urea is not fed to the reaction section, with the necessity however of maintaining a supply of NH3 to the section itself, are for example cases of NH3 used in various flushings to avoid possible clogging of the lines and allowing a rapid restart.

The same problem arises with greater significance during the emptying of one or more of the apparatuses of the reaction section of the plant by draining or sublimating the melamine contained therein and flushing for the complete emptying of the section(s) before proceeding with the opening of the apparatuses and their cleaning: in this case, as the emptying rate by sublimation is proportional to the quantity of ammonia fed, the quantity of ammonia used is certainly a significant quantity which can be higher than 1,000 kg/h. In addition, the emptying operation by sublimation of the melamine lasts for several days.

In recent years, in general, technologies that use urea for scrubbing gaseous streams have been preferred with respect to technologies that effect said scrubbing with water, as scrubbing with urea allows an off-gas stream to be obtained, to be recycled to the plants for the production of water-free urea. The absence of water in the off-gases is of particular interest as the energy consumption of the urea plant is consequently reduced.

The various technologies aimed at the production of melamine have therefore been updated in this sense, specifically in the light of the advantages associated with scrubbing the gaseous streams exiting from the reaction section of melamine production plants, with molten urea. After scrubbing the gaseous streams, the molten urea, is fed to the reactor for the production of melamine, thus ensuring, during the production running of the plant for the production of melamine, both the complete recovery of the melamine contained in the gaseous streams subjected to scrubbing, and also the export of anhydrous off-gases to the urea plant.

As previously specified, however, during the running of the plant, there are non-productive transitional phases during which, while all the sections downstream of the reaction section remain functioning, it is obligatory not to feed urea to the reactor, while continuing to feed ammonia, in order to maintain, for example, the necessary flushings or to empty the reaction section itself in order to carry out maintenance work.

During these transitional phases the gaseous streams leaving the reaction section no longer contain carbon dioxide, as the melamine formation reaction no longer takes place, but they are exclusively composed of ammonia and melamine in vapour phase.

In this situation, the molten urea used for scrubbing the gas phases can no longer be fed to the reactor and thus the quantity of renewal urea of the stream recirculating in the scrubbing column is lacking. Consequently, in order to avoid clogging of the scrubbing section due to the accumulation of melamine and/or the formation of by-products due to the degradation of urea, the gaseous streams must be sent to the urea plant without having been scrubbed or the urea, containing melamine, must be recycled to the urea plant or the urea must be drained in storage tanks to be subsequently reused when urea can once again be fed to the reactor.

The objective of the present invention is therefore to define an apparatus and a process for the treatment of the gaseous streams leaving the reaction section which overcome, in a particularly simple way, the above-mentioned drawbacks that arise when it is impossible to feed the reactor with the molten urea used for scrubbing the gaseous streams or, more generically, in the non-productive transitional phases of the melamine production process in which urea cannot be fed to the reactor, avoiding that the off-gases be sent to the urea plant.

These and other objectives are achieved by means of the apparatus and the process according to the present invention.

The present invention therefore relates to an apparatus suitable for the treatment of the gaseous streams leaving the reaction section in a plant for the production of melamine during phases wherein urea is not fed to the reaction section, said apparatus comprising a reaction section and a scrubbing section, wherein:-
- said reaction section comprises at least one reactor (R) and at least one post-reactor (PR);
- said scrubbing section (L) is suitable for scrubbing the gaseous stream coming from the reactor with urea;
- said reactor (R) is connected by means of an outlet duct of the gaseous stream (U1) to a line (T) suitable for transferring the gaseous stream from the reactor to the scrubbing section and suitable for transferring the gaseous stream from the post-reactor to the scrubbing section;
- said post reactor is connected by means of an outlet duct of the gaseous stream (U2) to said line (T) suitable for transferring the gaseous stream from the reactor to the scrubbing section and suitable for transferring the gaseous stream from the post-reactor to the scrubbing section;
- said transfer line (T), common to the reactor and post-reactor, being in turn connected to a transfer line (T1) suitable for transferring the gaseous streams leaving the reactor (R) and post-reactor (PR) to sections of the plant for the production of melamine other than the scrubbing section (L) and suitable for receiving said gaseous streams;
- a first shut-off valve (A), suitable for intercepting the transfer line (T) excluding said scrubbing section, is positioned on said transfer line (T), upstream of said scrubbing section;
- a second shut-off valve (B) is positioned on said transfer line (T1) suitable for transferring the gas phases leaving the reactor (R) and post-reactor (PR) to different sections of the plant for the production of melamine other than the scrubbing section (L) and suitable for receiving said gaseous streams.

The present invention further relates to a process for treating the gaseous streams leaving the reaction section of a plant for the production of melamine, wherein
a first gaseous stream leaving the reactor, comprising NH₃ and melamine in vapour phase, and a second gaseous stream leaving the post-reactor, comprising NH₃ and melamine in vapour phase, are combined and sent to sections of the plant for the production of melamine different from the scrubbing section (L) and suitable for receiving said gaseous streams, excluding a scrubbing step of said gaseous streams with molten urea when the melamine production process is in a non-productive transitional phase wherein no urea is fed to the reactor.

When the melamine production process is in said non-productive transitional phase wherein urea is not fed to the reactor, said gaseous streams are never sent to the urea plant.

Said transfer line (T1) suitable for transferring the gaseous streams leaving the reactor (R) and post-reactor (PR) to sections of the plant for the production of melamine other than the scrubbing section (L) and suitable for receiving said gaseous streams is not suitable for transferring the gaseous streams leaving the reactor (R) and post-reactor (PR) to the urea plant.

The apparatus and the process according to the present invention solve the drawbacks previously indicated in an extremely simple way through the temporary use of other sections of the melamine plant downstream of the reaction section, other than the scrubbing section, to which the gaseous streams exiting from the reactor and post reactor are sent, thus excluding the scrubbing step of the gaseous streams with urea. By means of simple but effective plant modifications, it is in fact possible to carry out the treatment of the gaseous stream leaving the reaction section, i.e. scrubbing, absorption and complete recovery, during the non-productive transitional phases of the melamine production process, in the absence of a supply of urea to the reactor; these modifications make it possible to connect the reaction section, or even more specifically the line that transfers the gaseous streams from the reaction section to the scrubbing section, to sections of the plant for the production of melamine suitable for receiving said gaseous streams and other than the scrubbing sections, which can vary according to the process used downstream of the reaction section.

Said sections other than the scrubbing sections and suitable for receiving said gaseous streams can be the section suitable for receiving the purified melamine leaving the post-reactor or sections suitable for receiving a gaseous stream containing ammonia such as, by way of non-limiting example, a device suitable for absorbing gaseous streams containing ammonia, in water, or a column suitable for separating ammonia from aqueous solutions.

In the present description, for the illustration of the figures, identical reference numbers or letters are used for indicating construction elements with the same function. Furthermore, for clarity of illustration, some references may not have been repeated in all of the figures.

Indications such as "vertical" and "horizontal", "upper" and "lower" (in the absence of other indications) should be read with reference to the assembly (or operating) conditions and referring to the normal terminology used in current language, wherein "vertical "indicates a direction substantially parallel to that of the force of gravity vector "g" and horizontal a direction perpendicular to it, coinciding with the "horizon direction".

In the present description, the term "upper", "lower" always refers to a position higher or lower with respect to the vertical direction.

In the present description, the term "downstream" refers to a position in an apparatus, means or pipe that follows with respect to the normal movement direction of the flow of the stream passing through a given apparatus, means or pipe.

In the present description, the term "upstream" refers to a position in an apparatus, means or pipe that precedes with respect to the normal movement direction of the flow of the stream passing through a given apparatus, means or pipe.

The apparatus according to the present invention preferably comprises a pressure-control system consisting of a control valve (CV2) positioned downstream of the second shut-off valve (B) on the transfer line (T1) and a pressure controller (PC2)), positioned upstream or downstream of said second shut-off valve (B).

In an alternative solution which is applied when the scrubbing section is at a lower pressure than the reaction section, the apparatus according to the present invention comprises a pressure-control system consisting of a control valve (CV1) and a pressure controller (PC1) positioned on the transfer line (T), common to the reactor and post-reactor, upstream of the two shut-off valves (A) and (B).

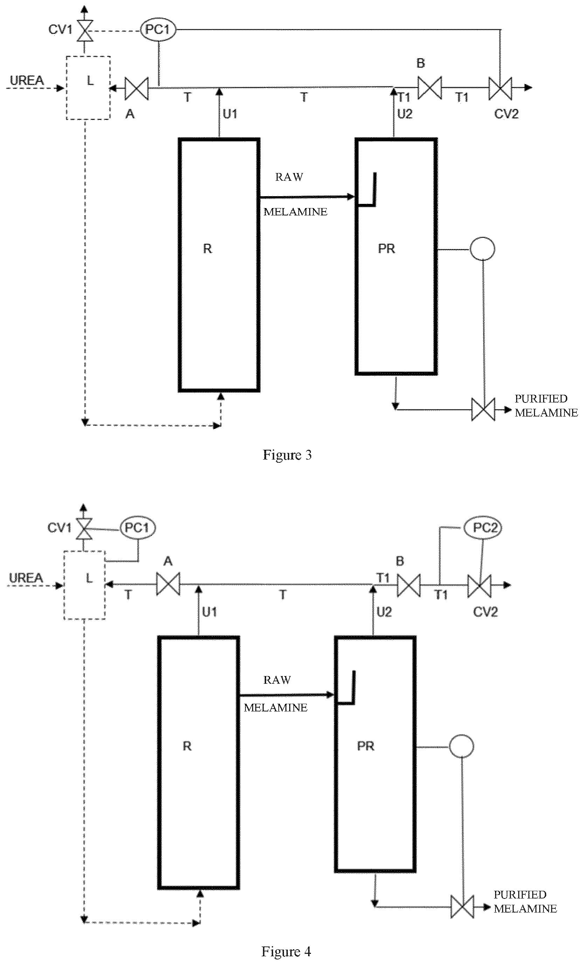

In the attached figures 1-5, figures 1 and 2 are representative of the reaction and scrubbing sections according to the state of the art in two different configurations, whereas figures 3-5 are representative of different embodiments of the apparatus according to the present invention .

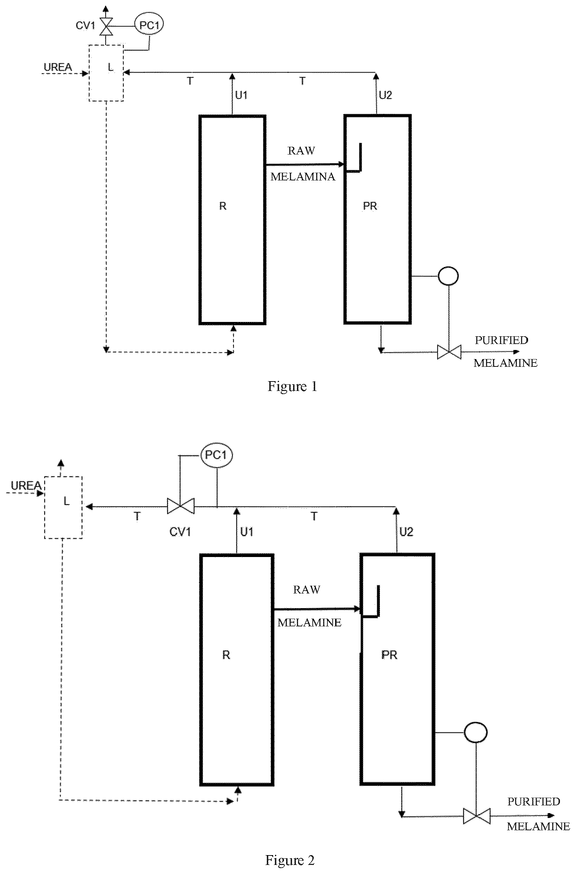

In the state of the art, the configuration of the reaction section together with the scrubbing section with urea is obtained using two different pressure-control methods:
(a) in equipressure (Figure 1)
(b) with differentiated pressure (Figure 2).

In case (a) represented in Figure 1, the pressure of the scrubbung section is equal to the pressure of the reaction section, in this case the pressure-control valve of the reaction section is positioned downstream of the scrubbing section. The pressure control in the reactor is in fact carried out by controlling the pressure of the off-gases leaving the scrubbing section.

In case (b) represented in Figure 2, the pressure of the scrubbing section is lower than the pressure of the reaction section, in this case the pressure-control valve of the reaction section is positioned upstream of the scrubbing section. The pressure control in the reactor is carried out by controlling the pressure of the gaseous stream leaving the reaction section.

The above-mentioned drawbacks are overcome by the apparatus and process according to the present invention by means of a simple but effective modification, capable of enabling treatment of the gaseous stream leaving the reaction section, which consists of scrubbing, absorption and total recovery of the gaseous stream, in sections other than the scrubbing section and suitable for receiving said gaseous streams within the same melamine plant, during the non-productive transitional phases wherein, as no urea is fed to the reactor, the melamine production reaction, which generates the gaseous stream containing CO₂, NH3 and melamine, does not take place, but a gas stream containing NH3 and melamine as described above, is obtained.

The solution according to the present invention brings substantial advantages to the urea/melamine plant complex as, in the absence of feeding of urea to the reactor, it allows the reaction section to be kept flushed with ammonia for as long as necessary, for example for the time necessary. for emptying the section by sublimation, which can last several days, treating the gaseous streams leaving the reaction section inside the melamine plant without interfering with the running conditions of the urea plant as it is not obliged to receive a CO₂-free stream.

When the off-gas stream from the melamine plant is free of CO₂, in order to rebalance the NH3/CO2 ratio required, the number of recyclings within the urea plant must be increased with a consequent increase in energy consumption. Furthermore, a CO₂-free stream, therefore rich in NH₃, makes it necessary to reduce the feeding rate of fresh NH3 to compensate for that fed in excess with the off-gas stream.

In a first embodiment of the apparatus according to the present invention, the insertion of said transfer line (T1) can be carried out as shown in figure 3: if the pressure of the scrubbing section is equal to the pressure of the reaction section, the pressure-control valve (CV1) of the reaction section is positioned downstream of the scrubbing section. If the pressure controller (PC1) receives the pressure signal from the reaction section (Fig. 3):
the transfer line (T), common to the reactor and post-reactor, is connected by means of a transfer line (T1) suitable for transferring the gas phases leaving the reactor (R) and the post-reactor (PR) to sections of the plant for the production of melamine other than the scrubbing section (L) and suitable for receiving said gaseous streams;
a first shut-off valve (A), suitable for allowing the exclusion of the scrubbing section, is positioned on said transfer line (T), upstream of said scrubbing section;
a second shut-off valve (B) and a pressure-control valve (CV2) are positioned on said transfer line (T1) suitable for transferring the gas phases leaving the reactor (R) and the post-reactor (PR) to sections of the plant for the production of melamine other than the scrubbing section (L) and suitable for receiving said gaseous streams,
the pressure-control valve (CV2) being positioned downstream of the second shut-off valve (B) on the transfer line (T1) .

The same pressure controller (PC1) can control the pressure-control valve (CV2) by means of a signal deviation function.

During the non-productive transitional phases of the melamine production process during which no urea is fed to the reactor and wherein the gaseous streams are consequently free of CO₂, the arrangement of the system will be as follows:
first valve (A) closed
second valve (B) open
PC1/CV2 pressure controller inserted.

The pressure control of the reaction section will therefore be obtained by means of the pressure controller (PC1), whereas the scrubbing section is isolated/excluded.

In a second embodiment of the apparatus according to the present invention, the insertion of said transfer line (T1) can be carried out as shown in figure 4:
if the pressure of the scrubbing section is equal to the pressure of the reaction section, a pressure-control valve (CV1, PC1) of the reaction section is positioned downstream of the scrubbing section. If the pressure controller receives the pressure signal from the same scrubbing section, the apparatus according to the present invention comprises (Figure 4):
the transfer line (T), common to the reactor and post-reactor, is connected by means of a transfer line (T1) suitable for transferring the gas phases leaving the reactor (R) and the post-reactor (PR) to sections of the plant for the production of melamine other than the scrubbing section (L) and suitable for receiving said gaseous streams;
a first shut-off valve (A), suitable for allowing the exclusion of the scrubbing section, is positioned on said transfer line (T), upstream of said scrubbing section;
a second shut-off valve (B) and a pressure-control unit (PC2/CV2) are positioned on said transfer line (T1) suitable for transferring the gas phases leaving the reactor (R) and the post-reactor (PR) to sections of the plant for the production of melamine other than the scfubbing section (L) and suitable for receiving said gaseous streams,
the pressure-control unit (PC2/CV2) being positioned downstream of the second shut-off valve (B) on the transfer line (T1).

During the non-productive transitional phases of the melamine production process during which no urea is fed to the reactor and wherein the gaseous streams are free of CO₂, the arrangement of the system will be as follows:
first valve (A) closed
second valve (B) open
pressure controller (PC2/CV2) inserted.

The pressure control of the reaction section will therefore be obtained by means of the pressure controller (PC2), whereas the scrubbing section is isolated/excluded.

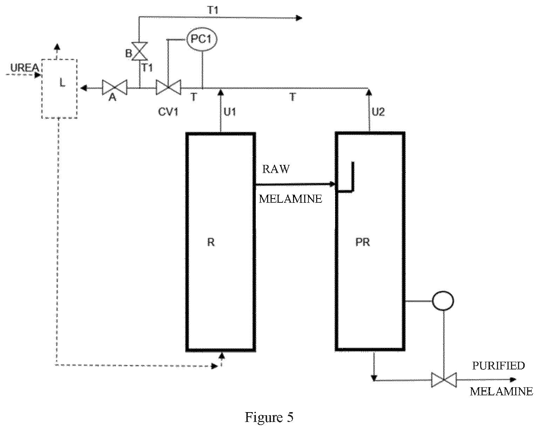

In a third embodiment of the apparatus according to the present invention, the insertion of said transfer line (T1) can be carried out as shown in figure 5: if the pressure of the scrubbing section is lower than the pressure of the reaction section, the pressure-control group (CV1, PC1) of the reaction section is positioned upstream of the scrubbing section.

If the pressure-control group (CV1,PC1) is positioned upstream of the scrubbing section, said group can also be used when sending the gaseous streams of the reaction into the sections other than the scrubbing section (L) and suitable for receiving said gaseous streams, as follows (Figure 5):
The transfer line (T), common to the reactor and post-reactor, is connected to a transfer line (T1) suitable for transferring the gas phases leaving the reactor (R) and the post-reactor (PR) to sections of the plant for the production of melamine other than the scrubbing section (L) and suitable for receiving said gaseous streams;
a first shut-off valve (A), suitable for allowing the exclusion of the scrubbing section, is positioned on said transfer line (T), upstream of said scrubbing section and downstream of the control valve (CV1);
a second shut-off valve (B) is positioned on said transfer line (T1) suitable for transferring the gas phases leaving the reactor (R) and the post-reactor (PR) to sections of the plant for the production of melamine other than the scrubbing section (L) and suitable for receiving said gaseous streams, said transfer line (T1) becoming detached from the transfer line (T) downstream of the control valve (CV1) and upstream of the first shut-off valve (A).

During the non-productive transitional phases, the arrangement of the system will be as follows:
first valve (A) closed
second valve (B) open
Pressure controller (PC1/CV1) inserted

The pressure control of the reaction section will therefore be effected by the pressure controller (PC1), whereas the scrubbing section is isolated/excluded.

The solution according to the present invention therefore allows, with few but substantial modifications, ammonia to be continuously sent to the reaction section even during long non-productive transitional phases, during which no urea is fed to the reactor, keeping the reaction section flushed and safe without the urea plant having to receive out-of-specification off-gases as it does not contain CO₂ and with the probable presence of melamine or without the melamine plant being compelled to store, for a long period of time, significant quantities of urea used for scrubbing, continuously subjected to degradation with various problems relating to its subsequent recovery.

## Claims

1. An apparatus suitable for the treatment of the gaseous streams leaving the reaction section in a plant for the production of melamine during phases in which there is no urea supply to the reaction section, said apparatus comprising a reaction section and a scrubbing section, wherein:
- said reaction section comprises at least one reactor (R) and at least one post-reactor (PR);
- said scrubbing section (L) is suitable for scrubbing the gaseous stream coming from the reactor with urea;
- said reactor (R) is connected by means of an outlet duct of the gaseous stream (U1) to a line (T) suitable for transferring the gaseous stream from the reactor to the scrubbing section and suitable for transferring the gaseous stream from the post-reactor to the scrubbing section;
- said post reactor is connected by means of an outlet duct of the gaseous stream (U2) to said line (T) suitable for transferring the gaseous stream from the reactor to the scrubbing section and suitable for transferring the gaseous stream from the post-reactor to the scrubbing section;
- said transfer line (T), common to the reactor and post-reactor, being in turn connected to a transfer line (T1) suitable for transferring the gaseous streams leaving the reactor (R) and the post-reactor (PR) to sections of the plant for the production of melamine different from the scrubbing section and suitable for receiving said gaseous streams;
- a first shut-off valve (A), suitable for intercepting the transfer line (T) excluding said scrubbing section, is positioned on said transfer line (T), upstream of said scrubbing section;
- a second shut-off valve (B) is positioned on said transfer line (T1) suitable for transferring the gas phases leaving the reactor (R) and the post-reactor (PR) to sections of the plant for the production of melamine other than the scrubbing section and suitable for receiving said gaseous streams.

2. The apparatus according to claim 1, wherein the sections other than the scrubbing section and suitable for receiving the gaseous streams are the section suitable for receiving the purified melamine leaving the post-reactor or sections suitable for receiving a gaseous stream containing ammonia such as for example a device suitable for absorbing gaseous streams containing ammonia, in water, or a column suitable for separating the ammonia from the aqueous solutions.

3. The apparatus according to any of the previous claims, wherein said apparatus comprises a pressure-control system consisting of a control valve (CV2) positioned downstream of the second shut-off valve (B) on the transfer line (T1) and a pressure controller (PC2/PC1), positioned upstream or downstream of said second shut-off valve (B).

4. The apparatus according to any of claims 1 or 2, wherein said apparatus comprises a pressure-control system consisting of a control valve (CV1) and a pressure controller (PC1) positioned on the transfer line (T), common to the reactor and post-reactor, upstream of the two shut-off valves (A) and (B).

5. A process for the treatment of the gaseous streams leaving the reaction section of a plant for the production of melamine, wherein
a first gaseous stream leaving the reactor, comprising NH3 and melamine in vapour phase, and a second gaseous stream leaving the post-reactor, comprising NH₃ and melamine in vapour phase, are combined and sent into sections of the plant for the production of melamine other than the scrubbing section and suitable for receiving said gaseous streams, excluding a scrubbing step of said gaseous streams with molten urea when the melamine production process is in a non-productive transitional phase during which urea is not fed to the reactor and excluding that said gaseous streams be sent to the urea plant.
